# EUROPÄISCHE PATENTANMELDUNG

(11) **EP 3 854 449 A1**
(43) Veröffentlichungstag der Anmeldung: **28.07.2021**
(21) Anmeldenummer: 20153795.8
(22) Anmeldetag: 27.01.2020
(51) Int. Cl.: A61N 1/05, A61N 1/39

(54) **IMPLANTIERBARES MEDIZINISCHES GERÄT ZUR STIMULATION EINES MENSCHLICHEN ODER TIERISCHEN HERZENS**

(71) Anmelder: BIOTRONIK SE & Co. KG, 12359 Berlin (DE)
(72) Erfinder: Dörr, Thomas, 12437 Berlin (DE); Weiss, Ingo, 12435 Berlin (DE); Friedrich, Michael, 14532 Kleinmachnow (DE); Kolberg, Gernot, 12161 Berlin (DE)
(74) Vertreter: Krämer, Thomas

(57) **Zusammenfassung**

Die Erfindung betrifft ein implantierbares medizinisches Gerät zur Stimulation eines menschlichen oder tierischen Herzens, mit einem Gehäuse, einer in dem Gehäuse angeordneten Stimulationseinheit zur Stimulation eines menschlichen oder tierischen Herzens (9), einer in dem Gehäuse angeordneten Detektionseinheit zur Detektion elektrischer Signale desselben Herzens (9) und einer ersten Elektrodenanordnung (1), die zumindest an die Stimulationseinheit angeschlossen ist, wobei die erste Elektrodenanordnung (1) einen ersten Elektrodenpol (2) aufweist, der zur Defibrillation des genannten Herzens (9) geeignet ist. Das Gerät zeichnet sich dadurch aus, dass der erste Elektrodenpol (2) ein erstes Elektrodensegment (3) und mindestens ein weiteres Elektrodensegment (4, 11) aufweist, wobei die einzelnen Elektrodensegmente (3, 4, 11) gegeneinander elektrisch isoliert sind, wobei das mindestens eine weitere Elektrodensegment (4, 11) von der Stimulationseinheit zusammen mit dem ersten Elektrodensegment (3) oder unabhängig von dem ersten Elektrodensegment (3) mit einem elektrischen Puls beaufschlagt werden kann.

## Beschreibung

Die vorliegende Erfindung betrifft ein implantierbares medizinisches Gerät zur Stimulation eines menschlichen oder tierischen Herzens gemäß dem Oberbegriff des Anspruchs 1, eine Elektrodenanordnung für ein implantierbares medizinisches Gerät zur Stimulation eines menschlichen oder tierischen Herzens sowie ein Verfahren zur Steuerung des Betriebs eines derartigen Geräts gemäß dem Oberbegriff des Anspruchs 12.

Zahlreiche aus dem Stand der Technik bekannte implantierbare medizinische Geräte zur Stimulation eines menschlichen oder tierischen Herzens, wie etwa Herzschrittmacher, implantierbare Kardioverter-Defibrillatoren (ICDs) oder Geräte zur kardialen Resynchronisationstherapie (CRTD) sind mit einer Elektrode ausgestattet, die einen oder mehrere Elektrodenpole aufweist. Dabei weisen die Elektrodenpole stets eine feste Länge auf, sodass die entsprechenden Elektroden hinreichend genau in einer anatomischen Struktur positioniert werden müssen, damit sie die gewünschte Wirkung entfalten können.

Herkömmliche Kardioverter-Defibrillatoren (ICDs) werden typischerweise in einem oberen Brustbereich implantiert und weisen eine Elektrode auf, die durch eine zum Herzen führende Vene, insbesondere durch die obere Hohlvene, in den rechten Ventrikel implantiert wird. Daher werden solche ICDs auch als transvenöse ICDs bezeichnet.

Darüber hinaus sind nicht-transvenöse ICDs bekannt, die auch als nicht-transvenös implantierbare Kardioverter-Defibrillatoren bezeichnet werden. Bei derartigen nichttransvenösen ICDs wird die Elektrode, mittels der im Behandlungsfall ein Defibrillationspuls abgegeben wird, nicht im Herzen, sondern außerhalb des Herzens positioniert. Um dennoch für eine möglichst gute Wirkung zu sorgen, muss das elektrische Feld, das zwischen der Elektrode und einer entsprechenden Gegenelektrode des ICDs verläuft, einen möglichst großen Bereich des Herzens erfassen. Aufgrund der anatomischen Unterschiede zwischen einzelnen Patienten ist dies naturgemäß sehr schwierig. Denn während einige Patienten ein besonders großes Herz haben, weisen andere Patienten lediglich ein kleines Herz auf. Mitunter werden unterschiedliche Elektroden angeboten, um unterschiedlich große Herzen in physiologisch sinnvoller Art und Weise stimulieren zu können. Aber auch dann ist eine besonders genaue Positionierung der Elektroden erforderlich, damit das gewünschte elektrische Feld einen großen Bereich des Herzens erfasst.

Der vorliegenden Erfindung liegt die Aufgabe zugrunde, ein implantierbares medizinisches Gerät zur Stimulation des menschlichen oder tierischen Herzens bereitzustellen, das eine größere Flexibilität bei der Implantation als die aus dem Stand der Technik bekannten Geräte zur Stimulation des menschlichen oder tierischen Herzens aufweist und gleichzeitig einen guten Stimulationserfolg ermöglicht.

Diese Aufgabe wird durch ein implantierbares medizinisches Gerät zur Stimulation eines menschlichen oder tierischen Herzens mit den Merkmalen des Anspruchs 1 gelöst. Ein derartiges Gerät weist ein Gehäuse auf, in dem eine Stimulationseinheit und eine Detektionseinheit positioniert sind. Die Stimulationseinheit dient zur Stimulation eines menschlichen oder tierischen Herzens, während die Detektionseinheit zur Detektion elektrischer Signale desselben Herzens vorgesehen ist. Ferner weist das Gerät eine Elektrodenanordnung auf, die zumindest an die Stimulationseinheit angeschlossen ist. Wenn mittels der Elektrodenanordnung auch elektrische Signale des Herzens detektiert werden sollen, ist die Elektrodenanordnung auch an die Detektionseinheit des Geräts angeschlossen. Die erste Elektrodenanordnung weist einen ersten Elektrodenpol auf, der zur Defibrillation des genannten Herzens geeignet ist. Das heißt, der Elektrodenpol ist dazu vorgesehen und eingerichtet, einen Defibrillationspuls abzugeben, mittels dessen eine kardiale Defibrillation erreicht werden kann.

Alternativ kann der genannte erste Elektrodenpol der ersten Elektrodenanordnung zur Stimulation des genannten Herzens geeignet sein. Das heißt, der Elektrodenpol ist in diesem Fall dazu vorgesehen und eingerichtet, einen Stimulationspuls abzugeben, mittels dessen eine kardiale Stimulation erreicht werden kann.

Erfindungsgemäß ist vorgesehen, dass die Größe bzw. Länge des ersten Elektrodenpols variabel ausgestaltet ist. Zu diesem Zweck weist der erste Elektrodenpol ein erstes Elektrodensegment und mindestens ein weiteres Elektrodensegment auf. Die einzelnen Elektrodensegmente sind gegeneinander elektrisch isoliert. Das heißt, das erste Elektrodensegment ist elektrisch gegenüber dem mindestens einen weiteren Elektrodensegment elektrisch isoliert. Wenn mehrere weitere Elektrodensegmente vorgesehen sind, sind auch diese gegeneinander elektrisch isoliert.

Das mindestens eine weitere Elektrodensegment kann von der Stimulationseinheit zusammen mit dem ersten Elektrodensegment oder unabhängig von dem ersten Elektrodensegment mit einem Defibrillationspuls oder einem Stimulationspuls beaufschlagt werden. Es ist also möglich, entweder nur das erste Elektrodensegment oder nur das mindestens eine weitere Elektrodensegment oder aber beide bzw. mehrere Elektrodensegmente derart anzusteuern, dass sie mit einem Defibrillationspuls oder einem Stimulationspuls beaufschlagt werden, den sie anschließend abgeben, um das betreffende zu behandelnde menschliche oder tierische Herz mit dem entsprechenden Defibrillationspuls oder dem entsprechenden Stimulationspuls zu beaufschlagen.

Mithin ist der erste Elektrodenpol als segmentierter Elektrodenpol ausgestaltet, dessen Länge und/oder dessen aktive Elektrodensegmente nach den anatomischen Gegebenheiten bei dem zu behandelnden Patienten und/oder in Abhängigkeit der konkreten Positionierung der ersten Elektrodenanordnung gegenüber dem zu stimulierenden Herzen angepasst werden kann. Es lassen sich also durch die erfindungsgemäß vorgesehene Lösung verschiedene Geometrien ein und desselben Elektrodenpols erreichen. Darüber hinaus ist optional die relative Lage des Elektrodenpols an die anatomischen Gegebenheiten unter Berücksichtigung der Implantationssituation der ersten Elektrodenanordnung anpassbar. Beispielsweise ist es möglich, nicht das erste Elektrodensegment mit einem Defibrillationspuls oder einem Stimulationspuls zu beaufschlagen, sondern lediglich ein oder mehrere des mindestens einen weiteren Elektrodensegments. Dann ist der erste Elektrodenpol gleichsam entlang der Längserstreckungsrichtung der ersten Elektrodenanordnung gegenüber einer Situation, in der lediglich der erste Elektrodenpol mit einem Defibrillationspuls oder einem Stimulationspuls beaufschlagt wird, verschoben.

Das erste Elektrodensegment und das mindestens eine weitere Elektrodensegment können auch als Elektrodenabschnitte oder als Elektrodensubpole bezeichnet werden. Im Unterschied zu unterschiedlichen Elektrodenpolen einer Elektrode sind derartige Elektrodensegmente stets einem einzigen Elektrodenpol zugehörig und sind in enger räumlicher Nähe zueinander ausgebildet.

Es können beispielsweise 1, 2, 3, 4, 5, 6, 7, 8, 9 oder 10 weitere Elektrodensegmente, insbesondere 1 bis 10, insbesondere 2 bis 9, insbesondere 3 bis 8, insbesondere 4 bis 7, insbesondere 5 bis 6 weitere Elektrodensegmente vorgesehen sein. Je größer die Anzahl der weiteren Elektrodensegmente, desto größer ist die Variabilität der Ausgestaltung des ersten Elektrodenpols hinsichtlich seiner Länge und hinsichtlich seiner relativen Position in der ersten Elektrodenanordnung. Die vorteilhaften Eigenschaften der vorliegend beschriebenen Erfindung lassen sich bereits mit einem einzigen weiteren Elektrodensegment realisieren.

In einer Variante befindet sich zwischen zwei benachbarten Elektrodensegmenten (also entweder zwischen dem ersten Elektrodensegment und einem benachbarten Elektrodensegment des mindestens einen weiteren Elektrodensegments oder zwischen zwei benachbarten weiteren Elektrodensegmenten) nur ein Isolator, nicht jedoch ein anderer Elektrodenpol. Auf diese Weise ist besonders einfach sichergestellt, dass die einzelnen Elektrodensegmente räumlich dicht beieinander angeordnet sind. Falls eine derartige räumlich dichte Anordnung der einzelnen Elektrodensegmente nicht gewünscht wird, ist es grundsätzlich auch denkbar, einen anderen Elektrodenpol (beispielsweise einen zur Detektion elektrischer Signale des Herzens eingesetzten Elektrodenpol) zwischen zwei benachbarten Segmenten des ersten Elektrodenpols anzuordnen.

In einer Ausgestaltung weisen die einzelnen angesteuerten Elektrodensegmente des ersten Elektrodenpols im Betrieb des implantierbaren medizinischen Geräts jeweils die gleiche Polarität auf. Das heißt, die einzelnen angesteuerten Elektrodensegmente werden auf die gleiche Polarität des abzugebenden Defibrillations- oder Stimulationspulses geschaltet. Dadurch kann besonders einfach sichergestellt werden, dass die einzelnen angesteuerten Elektrodensegmente tatsächlich als ein gemeinsamer Elektrodenpol wirken. In einer Variante ist es in dieser Ausgestaltung vorgesehen, dass die erste Elektrodenanordnung bereits hardwaretechnisch derart ausgestaltet ist, dass die einzelnen Elektrodensegmente nur mit gleicher Polarität angesteuert und eingesetzt werden können.

In einer Ausgestaltung handelt es sich bei dem implantierbaren medizinischen Gerät um einen nicht-transvenös implantierbaren Kardioverter-Defibrillator. Bei einem derartigen nicht-transvenös implantierbaren Kardioverter-Defibrillator lassen sich die erfindungsgemäßen Effekte besonders gut ausnutzen. Denn bei einem derartigen nichttransvenösen Kardioverter-Defibrillator ist die zur Defibrillation eingesetzte Elektrode (im vorliegenden Fall also die erste Elektrodenanordnung) außerhalb des zu behandelnden Herzens angeordnet. Ein elektrisches Feld, das im Fall der Abgabe von Defibrillationspulsen zwischen der ersten Elektrodenanordnung, die in diesem Fall als Defibrillationselektrode wirkt, und einer Gegenelektrode ausgebildet wird, muss sich einerseits über einen großen Bereich des zu behandelnden Herzens erstrecken und sollte andererseits keine wesentlichen Anteile aufweisen, die an dem Herzen vorbei verlaufen. Denn jeder Stromanteil, der nicht durch das zu behandelnde Herz geleitet wird, ist ein "verlorener" Strom, der zwar die Energieressourcen des nicht-transvenös implantierbaren Kardioverter-Defibrillators beansprucht, jedoch keinen Behandlungseffekt an dem zu behandelnden Herzen zeigt. Es ist im Falle eines nicht-transvenös implantierbaren Kardioverter-Defibrillator also besonders wichtig, dass der erste Elektrodenpol der ersten Elektrodenanordnung besonders vorteilhaft gegenüber dem zu behandelnden Herzen angeordnet ist, um ein wirkungsvolles elektrisches Feld aufbauen zu können.

Ein elektrisches Feld, das hingegen im Fall der Abgabe von Stimulationspulsen zwischen der ersten Elektrodenanordnung, die in diesem Fall als Stimulationselektrode wirkt, und einer Gegenelektrode ausgebildet wird, muss sich einerseits über einen spezifischen Bereich des zu behandelnden Herzens erstrecken und sollte andererseits eine hohe Stromdichte aufweisen, wenn es das Herz zu einer gezielten Kontraktion anregen soll. In diesem Fall ist daher nur ein Elektrodensegment des ersten Elektrodenpols der ersten Elektrodenanordnung anzusteuern.

Wenn die erste Elektrodenanordnung im Körper des Patienten implantiert ist, ist eine Korrektur ihrer Lage innerhalb des Körpers des Patienten regelmäßig nicht mehr möglich. Wenn dann aber unterschiedliche Elektrodensegmente des ersten Elektrodenpols angesteuert werden können, kann eine virtuelle Lageänderung des ersten Elektrodenpols erfolgen. Somit lässt sich die effektive Lage des ersten Elektrodenpols im Hinblick auf die anatomischen Gegebenheiten auch nach der Implantation optimieren, um so ein möglichst günstiges elektrisches Feld zur Gegenelektrode aufzubauen.

In einer Ausgestaltung weist das erste Elektrodensegment eine größere Länge als das mindestens eine weitere Elektrodensegment auf. Diese Ausgestaltung ist insbesondere dann sinnvoll, wenn vorgesehen ist, das erste Elektrodensegment stets anzusteuern. Dann kann durch das eine weitere Elektrodensegment im Bedarfsfall eine Verlängerung des ersten Elektrodenpols durchgeführt werden. Beispielsweise kann das mindestens eine weitere Elektrodensegment eine Länge aufweisen, die 10 % bis 90 %, insbesondere 20 % bis 80 %, insbesondere 30 % bis 70 %, insbesondere 40 % bis 60 %, insbesondere 25 % bis 50 % der Länge des ersten Elektrodensegment entspricht.

In einer anderen Ausgestaltung weisen alle Elektrodensegmente die gleiche Länge auf.

Wie bereits erläutert, ist typischerweise eine Gegenelektrode zu der ersten Elektrodenanordnung vorgesehen, wobei dann zwischen der ersten Elektrodenanordnung und der Gegenelektrode ein elektrisches Feld aufgebaut wird, in dem die Defibrillationspulse zur Defibrillation des zu behandelnden Herzens wirken. Als Gegenelektrode kann dabei insbesondere das Gehäuse des implantierbaren medizinischen Geräts vorgesehen sein. Wenn das implantierbare medizinische Gerät als nicht-transvenös implantierbarer Kardioverter-Defibrillator ausgestaltet ist, wird das entsprechende Gehäuse typischerweise zwischen dem Musculus latissimus dorsi (Großer Rückenmuskel) und dem Musculus serratus anterior (vorderer Sägezahnmuskel) implantiert. Die erste Elektrodenanordnung wird dann typischerweise rechts des zu behandelnden Herzens implantiert, sodass ein zwischen der ersten Elektrodenanordnung und dem Gehäuse aufgebautes elektrisches Feld durch das zu behandelnde Herz verläuft.

In einer Ausgestaltung ist indes vorgesehen, dass das implantierbare medizinische Gerät eine von dem Gehäuse verschiedene zweite Elektrodenanordnung aufweist, die einen zweiten Elektrodenpol aufweist, der als Gegenelektrode für den ersten Elektrodenpol geeignet ist. In einer derartigen Ausgestaltung würde die erste Elektrodenanordnung auf einer ersten Seite des zu behandelnden Herzens und die zweite Elektrodenanordnung auf einer zweiten Seite des zu behandelnden Herzens implantiert werden. Das zwischen den beiden Elektrodenanordnungen wirkende elektrische Feld würde dann auch durch das zu behandelnde Herz hindurch verlaufen.

Die erste Elektrodenanordnung und/oder die zweite Elektrodenanordnung können beispielsweise subkutan oder substernal implantiert sein.

In einer Ausgestaltung weist der zweite Elektrodenpol ein erstes Elektrodensegment und mindestens ein weiteres Elektrodensegment auf. Die einzelnen Elektrodensegmente sind dabei gegeneinander elektrisch isoliert. Das mindestens eine weitere Elektrodensegment kann in dieser Ausgestaltung zusammen mit dem ersten Elektrodensegment oder unabhängig von dem ersten Elektrodensegment als Gegenelektrode für den ersten Elektrodenpol der ersten Elektrodenanordnung dienen. Es ist also möglich, auch die als Gegenelektrode fungierende zweite Elektrodenanordnung mit einem segmentierten Elektrodenpol auszugestalten.

In einer Ausgestaltung sind die erste Elektrodenanordnung und die zweite Elektrodenanordnung baugleich ausgestaltet. Durch eine derartige Realisierung reduzieren sich die Herstellungskosten, da keine unterschiedlichen Elektrodenanordnungen mehr hergestellt werden müssen. Vielmehr lässt sich ein bestimmter Typ einer Elektrodenanordnung sowohl als erste Elektrodenanordnung für die Defibrillation des zu behandelnden Herzens und als zweite Elektrodenanordnung, welche als Gegenelektrode für die erste Elektrodenanordnung dient, einsetzen.

In einer Ausgestaltung weist die erste Elektrodenanordnung einen weiteren Elektrodenpol auf, der entweder proximal oder distal von dem ersten Elektrodenpol angeordnet ist. In gleicher Weise kann die zweite Elektrodenanordnung mit einem weiteren Elektrodenpol ausgestattet sein, der proximal oder distal von dem zweiten Elektrodenpol angeordnet ist.

Es ist auch möglich, einen weiteren Elektrodenpol proximal des ersten Elektrodenpols und einen weiteren Elektrodenpol distal des ersten Elektrodenpols anzuordnen. In gleicher Weise kann jeweils ein weiterer Elektrodenpol proximal und distal des zweiten Elektrodenpols angeordnet werden. Es ist also möglich, die Elektrodenanordnung als mehrpolige Elektrodenanordnung auszugestalten.

In einer Ausgestaltung dient der weitere Elektrodenpol der ersten Elektrodenanordnung und/oder der zweiten Elektrodenanordnung zur Detektion elektrischer Signale des zu behandelnden Herzens. Es ist also möglich, eine Stimulationselektrode bzw. Defibrillationselektrode (nämlich in Form des ersten Elektrodenpols) und eine Detektionselektrode (nämlich in Form eines weiteren Elektrodenpols) in ein und derselben Elektrodenanordnung zu realisieren. Wenn ein zur Detektion vorgesehener Elektrodenpol in der entsprechenden Elektrodenanordnung vorgesehen ist, ist die Elektrodenanordnung nicht nur an die Stimulationseinheit des implantierbaren medizinischen Geräts zur Stimulation des menschlichen oder tierischen Herzens angeschlossen, sondern auch an die Detektionseinheit dieses Geräts.

Der Anschluss der Elektrodenanordnung an das Gehäuse bzw. an die Stimulationseinheit und/oder die Detektionseinheit des implantierbaren medizinischen Geräts kann in herkömmlicher Weise mittels handelsüblicher Stecker, beispielsweise mittels eines DF4-Steckers, erfolgen. Andere Steckverbindungen, die eine elektrisch leitende Verbindung zwischen Leitern, die im Innern der Elektrodenanordnung verlaufen und zur Versorgung der einzelnen Elektrodensegmente mit elektrischen Impulsen dienen, und der Stimulationseinheit und/oder der Detektionseinheit herstellen, sind ebenfalls denkbar.

Ein Aspekt der vorliegenden Erfindung betrifft eine Elektrodenanordnung für ein implantierbares medizinisches Gerät zur Stimulation eines menschlichen oder tierischen Herzens, insbesondere ein implantierbares medizinisches Gerät gemäß den vorherigen Erläuterungen (jedoch ohne angeschlossene erste Elektrodenanordnung). Die Elektrodenanordnung weist einen ersten Elektrodenpol auf, der zur Defibrillation des genannten Herzens geeignet ist. Erfindungsgemäß ist vorgesehen, dass der erste Elektrodenpol ein erstes Elektrodensegment und mindestens ein weiteres Elektrodensegment aufweist. Die einzelnen Elektrodensegmente sind dabei gegeneinander elektrisch isoliert. Das mindestens eine weitere Elektrodensegment kann von einer Stimulationseinheit eines implantierbaren medizinischen Geräts zusammen mit dem ersten Elektrodensegment oder unabhängig von dem ersten Elektrodensegment mit einem Defibrillationspuls oder einem Stimulationspuls beaufschlagt werden.

Diese Elektrodenanordnung ist insbesondere dafür vorgesehen, an ein implantierbares medizinisches Gerät gemäß den vorherigen Erläuterungen (jedoch noch ohne erste Elektrodenanordnung) als erste Elektrodenanordnung angeschlossen zu werden.

Ein Aspekt der vorliegenden Erfindung betrifft ein Verfahren zur Steuerung des Betriebs eines implantierbaren medizinischen Geräts entsprechend den vorherigen Erläuterungen. Wie dargelegt, weist das implantierbare medizinische Gerät ein Gehäuse sowie eine Stimulationseinheit und eine Detektionseinheit in dem Gehäuse auf. Die Stimulationseinheit dient zur Stimulation eines menschlichen oder tierischen Herzens und die Detektionseinheit dient zur Detektion elektrischer Signale desselben Herzens. Ferner weist das Gerät eine erste Elektrodenanordnung auf, die zumindest an die Stimulationseinheit angeschlossen ist. Die erste Elektrodenanordnung wiederum weist einen ersten Elektrodenpol auf, der zur Defibrillation und/oder zur Stimulation des genannten Herzens geeignet ist. Der erste Elektrodenpol weist ein erstes Elektrodensegment und mindestens ein weiteres Elektrodensegment auf. Die einzelnen Elektrodensegmente sind gegeneinander elektrisch isoliert.

Das Verfahren zeichnet sich dadurch aus, dass die nachfolgend erläuterten Schritte durchgeführt werden.

Zunächst wird vorgegeben, welche Elektrodensegmente im Fall einer gewünschten Defibrillation oder einer Stimulation des Herzens eingesetzt werden sollen.

Anschließend werden die entsprechend der vorherigen Vorgabe ausgewählten Elektrodensegmente so verschaltet, dass die Stimulationseinheit die ausgewählten Elektrodensegmente mit einem Stimulationspuls beaufschlagen kann. Ein derartiger Stimulationspuls wird dann nachfolgend von den beaufschlagten Elektrodensegmenten des ersten Elektrodenpols abgegeben.

Die Vorgabe, welche Elektrodensegmente eingesetzt werden sollen, und das Verschalten werden gemäß dem erfindungsgemäß beanspruchten Verfahren so lange beibehalten, bis eine neue Vorgabe, welche Elektrodensegmente im Fall einer gewünschten Defibrillation oder Stimulation des genannten Herzens eingesetzt werden soll, gemacht wird.

Durch dieses Verfahren ist es auf besonders einfache Weise möglich, beispielsweise durch eine einmalige Definition, welche Elektrodensegmente zur Defibrillation oder zur Stimulation einzusetzen sind, eine entsprechende Ansteuerung dieser Elektrodensegmente zu erreichen. Diese Einstellung muss typischerweise nur einmal während der Lebensdauer des implantierbaren medizinischen Geräts durchgeführt werden, nämlich unmittelbar nach der Implantation des Geräts. Denn dann steht die relative Lage der ersten Elektrodenanordnung zu dem zu stimulierenden Herz fest. Typischerweise verändert sich diese relative Anordnung nicht. Falls es doch zu einer Elektrodendislokation kommen sollte, wäre diese aber problemlos ausgleichbar, ohne die Elektrodenanordnung selbst neu zu positionieren. Denn dann kann eine neue Vorgabe, welche Elektrodensegmente im Fall einer gewünschten Defibrillation oder Stimulation des Herzens eingesetzt werden sollen, gemacht werden. Nachfolgend werden dann die der neuen Vorgabe entsprechenden Elektrodensegmente so verschaltet, dass die Stimulationseinheit die ausgewählten Elektrodensegmente mit einem Defibrillationsspuls oder einem Stimulationspuls beaufschlagen kann.

Um die für eine Defibrillation auszuwählenden Elektrodensegmente zu bestimmen, kann beispielsweise eine Impedanzmessung oder eine Defibrillationserfolgsmessung zwischen der ersten Elektrodenanordnung und der entsprechenden Gegenelektrode des implantierbaren medizinischen Geräts durchgeführt werden. Mittels einer derartigen Impedanz- oder Defibrillationserfolgsmessung kann besonders einfach und verlässlich bestimmt werden, welcher Erfolg bei einer tatsächlichen Defibrillation des Herzens erwartet werden kann. Im Rahmen einer derartigen Testmessung können verschiedene Elektrodensegmente aktiviert oder deaktiviert werden, um die für eine Stimulation des zu behandelnden Herzens optimale Einstellung zu ermitteln.

In einer Variante erfolgt das Verschalten der ausgewählten Elektrodensegmente in elektronischer Weise. Zu diesem Zweck können elektronische, programmierbare Schalter vorgesehen sein, die einzelne Elektrodensegmente zu- bzw. abschalten können, die also für eine Aktivierung oder Deaktivierung einzelner Elektrodensegmente sorgen. Grundsätzlich kann für eine derartige elektronische Schaltung auch ein Multiplexer eingesetzt werden.

In einer weiteren Ausgestaltung erfolgt das Verschalten in ferngesteuerter Weise. Dann kann über einen Fernzugriff auf das implantierbare medizinische Gerät ein Steuerungsbefehl gesendet werden, der für ein Verschalten der entsprechenden Elektrodensegmente mit der Stimulationseinheit, insbesondere für ein elektronisches Verschalten, sorgt. Ein derartiger Fernzugriff kann im Rahmen der Ersteinrichtung des implantierbaren medizinischen Geräts oder im Rahmen nachfolgender (ggf. wiederkehrender) Routineuntersuchungen durchgeführt werden.

Ein Aspekt der vorliegenden Erfindung betrifft ein Verfahren zur Behandlung eines menschlichen oder tierischen Patienten, der eine solche Behandlung benötigt. Die Behandlung erfolgt mittels eines implantierbaren medizinischen Geräts gemäß den obigen Erläuterungen. Wie bereits dargelegt, weist das implantierbare medizinische Gerät ein Gehäuse sowie eine Stimulationseinheit und eine Detektionseinheit in dem Gehäuse auf. Die Stimulationseinheit dient zur Stimulation des Herzens des Patienten und die Detektionseinheit dient zur Detektion elektrischer Signale des Herzens des Patienten. Ferner weist das Gerät eine erste Elektrodenanordnung auf, die zumindest an die Stimulationseinheit angeschlossen ist. Die erste Elektrodenanordnung wiederum weist einen ersten Elektrodenpol auf, der zur Defibrillation des Herzens des Patienten geeignet ist. Der erste Elektrodenpol weist ein erstes Elektrodensegment und mindestens ein weiteres Elektrodensegment auf. Die einzelnen Elektrodensegmente sind gegeneinander elektrisch isoliert.

Das Behandlungsverfahren weist die nachfolgend erläuterten Schritte auf.

Zunächst wird vorgegeben, welche Elektrodensegmente im Fall einer gewünschten Defibrillation des Herzens oder Stimulation des Herzens des Patienten eingesetzt werden sollen. Diese Vorgabe kann beispielsweise auf der Grundlage einer vorherigen Testmessung erfolgen, die als Impedanzmessung, als Defibrillationserfolgsmessung oder als Stimulationserfolgsmessung ausgestaltet sein kann. Diese Testmessung kann in einer Variante ein Teil des beanspruchten Behandlungsverfahrens sein.

Auf der Grundlage der getroffenen Vorgabe erfolgt anschließend eine Verschaltung der ausgewählten Elektrodensegmente mit der Stimulationseinheit. Die Stimulationseinheit ist dann in der Lage, die ausgewählten Elektrodensegmente mit einem Stimulationspuls (Defibrillationspuls) zu beaufschlagen, während die nicht ausgewählten und somit nicht mit der Stimulationseinheit verschalteten Elektrodensegmente nicht mit einem Stimulationspuls beaufschlagt werden können.

Anschließend wird ein Defibrillationspuls oder ein Stimulationspuls an alle ausgewählten verschalteten Elektrodensegmente übermittelt, damit das Herz des Patienten über alle ausgewählten und verschalteten Elektrodensegmente mit diesem Defibrillationspuls oder Stimulationspuls beaufschlagt werden kann. Mithin werden alle ausgewählten und verschalteten Elektrodensegmente zur Abgabe des Defibrillationspulses eingesetzt, während die nicht ausgewählten und damit auch nicht verschalteten Elektrodensegmente nicht zur Abgabe des Defibrillationspulses eingesetzt werden. Auf diese Weise lässt sich ein an die anatomischen Besonderheiten des jeweiligen Patienten optimiertes elektrisches Feld für die abgegebenen Defibrillationspulse realisieren.

Sämtliche Varianten und Ausgestaltungen des beschriebenen implantierbaren medizinischen Geräts sind in beliebiger Weise miteinander kombinierbar und einzeln oder in beliebiger Kombination auf die beschriebene Elektrodenanordnung oder eines der beschriebenen Verfahren übertragbar. Ferner sind alle Varianten und Ausgestaltungen der beschriebenen Elektrodenanordnung miteinander kombinierbar und einzeln oder in beliebiger Kombination auf das implantierbare medizinische Gerät oder eines der beschriebenen Verfahren übertragbar. In gleicher Weise können die Varianten und Ausgestaltungen der einzelnen Verfahren in beliebiger Weise miteinander kombiniert werden und einzeln oder in beliebiger Kombination auf das jeweils andere Verfahren oder auf das beschriebene implantierbare medizinische Gerät oder die beschriebene implantierbare Elektrodenanordnung übertragen werden.

Weitere Details von Ausgestaltungen der vorliegenden Erfindung werden nachfolgend anhand von Ausführungsbeispielen und Figuren näher erläutert. Es zeigen:
- Figur 1A: eine schematische Darstellung eines ersten Ausführungsbeispiels einer Elektrodenanordnung für einen implantierbaren Kardioverter-Defibrillator;
- Figur 1B: eine schematische Darstellung der Elektrodenanordnung der Figur 1A in einer ersten Implantationssituation;
- Figur 1C: eine schematische Darstellung der Elektrodenanordnung der Figur 1A in einer zweiten Implantationssituation;
- Figur 2A: eine schematische Darstellung eines zweiten Ausführungsbeispiels einer Elektrodenanordnung für einen implantierbaren Kardioverter-Defibrillator;
- Figur 2B: eine schematische Darstellung der Elektrodenanordnung der Figur 2A in einer ersten Implantationssituation und
- Figur 2C: eine schematische Darstellung der Elektrodenanordnung der Figur 2A in einer zweiten Implantationssituation.

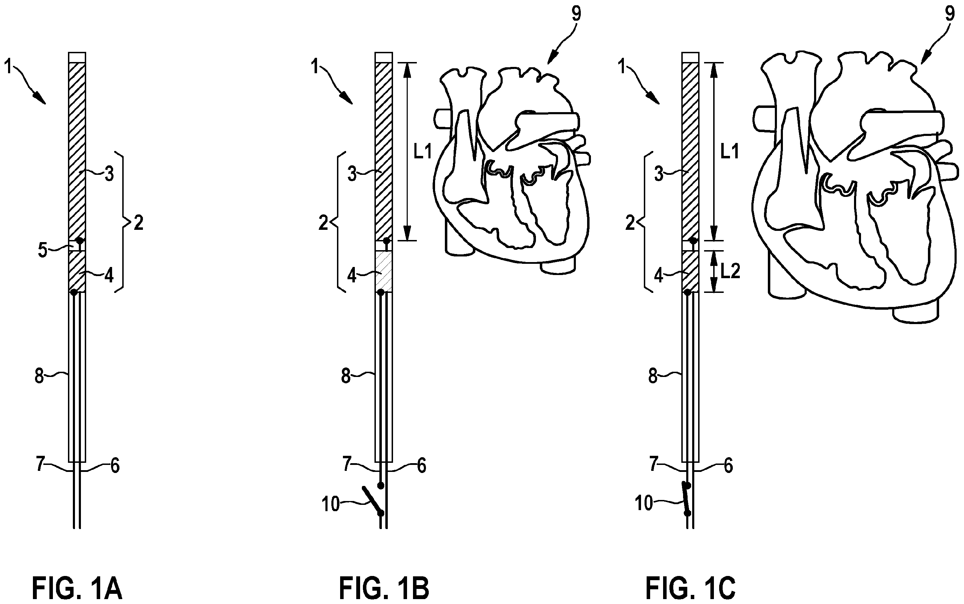

Die Figur 1A zeigt ein erstes Ausführungsbeispiel einer Elektrodenanordnung 1, die zum Anschluss an einen implantierbaren Kardioverter-Defibrillator als implantierbares medizinisches Gerät vorgesehen und eingerichtet ist. Die Elektrodenanordnung 1 weist einen ersten Elektrodenpol 2 auf, der ein größeres erstes Elektrodensegment 3 und ein kleineres zweites Elektrodensegment 4 aufweist. Zwischen dem ersten Elektrodensegment 3 und dem zweiten Elektrodensegment 4 ist ein Isolator 5 angeordnet, der das erste Elektrodensegment 3 elektrisch von dem zweiten Elektrodensegment 4 trennt.

Eine erste Elektrodenleitung 6 ist mit dem ersten Elektrodensegment 3 in elektrisch leitfähiger Weise verbunden. In gleicher Weise ist eine zweite Elektrodenleitung 7 elektrisch leitfähig mit dem zweiten Elektrodensegment 4 verbunden. Mittels der ersten Elektrodenleitung 6 und der zweiten Elektrodenleitung 7 lassen sich folglich das erste Elektrodensegment 3 und das zweite Elektrodensegment 4 unabhängig voneinander ansteuern.

Die erste Elektrodenleitung 6 und die zweite Elektrodenleitung 7 verlaufen in einem elektrisch isolierenden Elektrodenschlauch 8 der Elektrodenanordnung 1.

Sowohl das erste Elektrodensegment 3 als auch das zweite Elektrodensegment 4 sind als Schockwendel ausgestaltet. Mit ihnen kann ein Schockpuls zur Defibrillation eines menschlichen oder tierischen Herzens abgegeben werden.

In einem in der Figur 1A nicht dargestellten Defibrillationsgenerator ist ein elektronischer Schalter ausgebildet, mit dem ein Stromfluss durch die zweite Elektrodenleitung 7 zugelassen oder unterbrochen werden kann. Auf diese Weise ist es möglich, das zweite Elektrodensegment 4 zu dem ersten Elektrodensegment 3 zuzuschalten (wenn der elektronische Schalter geschlossen ist) bzw. von dem ersten Elektrodensegment 3 abzuschalten (wenn der elektronische Schalter geöffnet ist).

Die Figur 1B zeigt eine Implantationssituation der Elektrodenanordnung 1 im Körper eines Patienten, der ein eher kleines Herz 9 hat. Dabei werden in dieser und in allen folgenden Figuren vergleichbare Elemente stets mit denselben Bezugszeichen versehen.

Das erste Elektrodensegment 3 des ersten Elektrodenpols 2 weist eine Länge L1 auf, die im Wesentlichen der Höhe des Herzens 9 entspricht. Daher ist es zur Abgabe eines Defibrillationspulses von der Elektrodenanordnung 1 an das Herz 9 vollkommen ausreichend, wenn das erste Elektrodensegment 3 aktiv ist. Daher ist der in dem Defibrillationsgenerator ausgebildete und in der Figur 1B schematisch dargestellte elektronische Schalter 10 in diesem Ausführungsbeispiel geöffnet. Dann kann lediglich das erste Elektrodensegment 3 zur Abgabe eines Defibrillationspulses eingesetzt werden, nicht jedoch das zweite Elektrodensegment 4.

Wenn die gleiche Elektrodenanordnung 1 zur Stimulation eines größeren Herzens 9 eingesetzt werden soll, stellt sich die Situation anders dar. Dies ist in der Figur 1C dargestellt. Um das größere Herz 9 effektiv mit einem Defibrillationspuls beaufschlagen zu können, würde die Länge L1 des ersten Elektrodensegments 3 nicht ausreichend sein. Daher wird das zweite Elektrodensegment 4 durch Schließen des elektronischen Schalters 10 zum ersten Elektrodensegment 3 zugeschaltet. Dann steht die Summe aus der Länge L1 des ersten Elektrodensegments 3 und der Länge L2 des zweiten Elektrodensegments 4 zur Abgabe eines Defibrillationspulses an das Herz 9 zur Verfügung. Die Kombination des ersten Elektrodensegments 3 und des zweiten Elektrodensegments 4 sorgt mithin auch bei einem größeren Herzen für eine ausreichende Defibrillationswirkung. Es ist somit nicht erforderlich, im Falle der Implantation eines implantierbaren Kardioverter-Defibrillators unterschiedliche Elektrodenanordnungen vorrätig zu halten, die nach den anatomischen Gegebenheiten des zu behandelnden Patienten ausgewählt werden. Vielmehr kann eine einzige Elektrodenanordnung 1 vorgehalten werden, die elektronisch an die jeweiligen anatomischen Gegebenheiten angepasst werden kann.

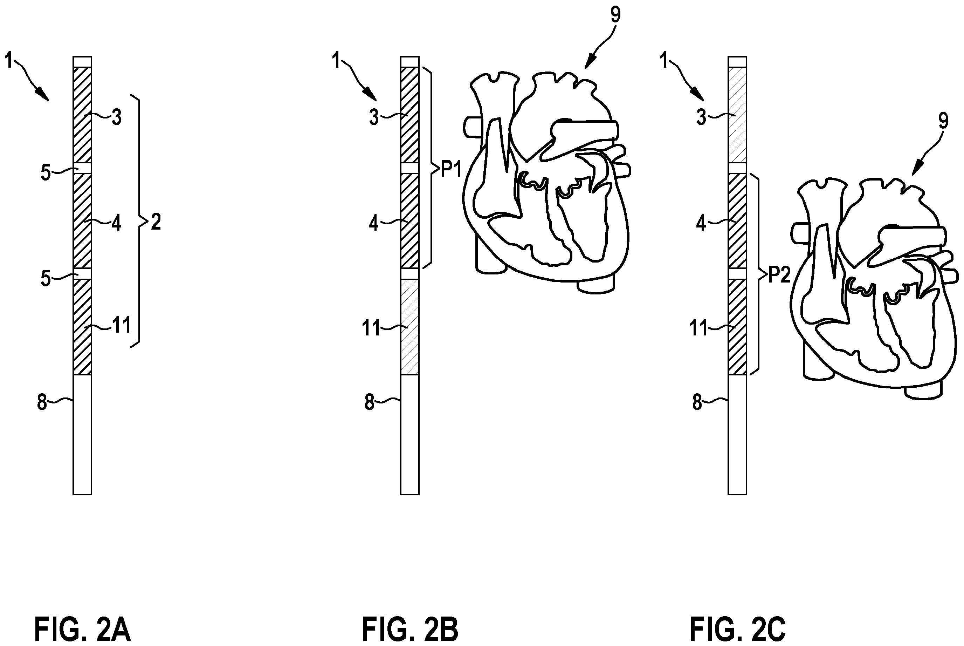

Die Figur 2A zeigt ein zweites Ausführungsbeispiel einer Elektrodenanordnung 1 in schematischer Darstellung. Auch diese Elektrodenanordnung 1 weist einen ersten Elektrodenpol 2 auf, der jedoch - anders als im Ausführungsbeispiel der Figuren 1A bis 1C - nicht zwei, sondern drei Elektrodensegmente aufweist, nämlich ein erstes Elektrodensegment 3, ein zweites Elektrodensegment 4 und ein drittes Elektrodensegment 11.

Zwischen dem ersten Elektrodensegment 3 und dem zweiten Elektrodensegment 4 sowie zwischen dem zweiten Elektrodensegment 4 und dem dritten Elektrodensegment 11 ist jeweils ein Isolator 5 angeordnet, der die entsprechenden Elektrodensegmente elektrisch voneinander isoliert. Innerhalb eines isolierenden Elektrodenschlauchs 8 verlaufen voneinander getrennte Zuleitungen zu den einzelnen Elektrodensegmenten 3, 4, 11, die jedoch in der Figur 2A nicht gesondert dargestellt sind. Die einzelnen Elektrodensegmente 3, 4, 11 sind vollkommen unabhängig voneinander ansteuerbar und zur Abgabe eines Defibrillationspulses einsetzbar. Dies wird in den Figuren 2B und 2C näher erläutert.

Die Figur 2B zeigt die Elektrodenanordnung 1 in einem ersten Implantationszustand neben dem Herzen 9 eines Patienten. Die Elektrodenanordnung 1 ist gegenüber dem Herzen 9 derart positioniert, dass eine gute Defibrillationsleistung erzielt werden kann, wenn das erste Elektrodensegment 3 und das zweite Elektrodensegment 4 zur Defibrillation ausgewählt und durch eine entsprechende Verschaltung von einer Stimulationseinheit eines implantierbaren Kardioverter-Defibrillators, an den die Elektrodenanordnung 1 angeschlossen ist, mit einem Defibrillationspuls versorgt werden können. Wenn eine Stimulationseinheit des implantierbaren Kardioverter-Defibrillators nun einen Defibrillationspuls abgibt, so wird dieser an das erste Elektrodensegment 3 und das zweite Elektrodensegment 4 geleitet, die den Defibrillationspuls anschließend gemeinsam an das Herz 9 abgeben. Somit wird in einer ersten Position P1 durch das erste Elektrodensegment 3 und das zweite Elektrodensegment 4 ein aktiver Elektrodenpol gebildet.

Die Figur 2C zeigt die Elektrodenanordnung 1 in einem weiteren Implantationszustand. In diesem Implantationszustand ragt die Elektrodenanordnung 1 oben etwas über das zu stimulierende Herz 9 hinaus. Daher wäre es ungünstig, wenn das erste Elektrodensegment 3 und das zweite Elektrodensegment 4 an der Position P1 (vergleiche Figur 2B) einen aktiven Elektrodenpol bilden würden. Folglich ist in dem Ausführungsbeispiel der Figur 2C das erste Elektrodensegment 3 nicht zur Abgabe eines Defibrillationspulses ausgewählt und somit auch nicht mit der Stimulationseinheit des implantierbaren Kardioverter-Defibrillators, an den die Elektrodenanordnung 1 angeschlossen ist, verschaltet. Vielmehr wurden das zweite Elektrodensegment 4 und das dritte Elektrodensegment 11 zur Bildung eines aktiven Elektrodenpols an einer zweiten Position P2 ausgewählt und mit der Stimulationseinheit verschaltet. Folglich wird ein von der Stimulationseinheit an die Elektrodenanordnung überführter Defibrillationspuls an der Position P2 gemeinsam von dem zweiten Elektrodensegment 4 und dem dritten Elektrodensegment 11 abgegeben. Damit kann auch in dieser Implantationssituation eine wirksame Defibrillation des zu behandelnden Herzens 9 erreicht werden.

Auch beim Ausführungsbeispiel der Figuren 2A, 2B und 2C sind die einzelnen Elektrodensegmente 3, 4, 11 als Schockwendeln ausgestaltet. Dabei sind die einzelnen Elektrodensegmente gleich lang. Als Gegenelektrode für die Elektrodenanordnung 1 wird sowohl beim Ausführungsbeispiel der Figuren 1A bis 1C als auch beim Ausführungsbeispiel der Figuren 2A bis 2C das Gehäuse des entsprechenden Kardioverter-Defibrillators verwendet. Ein zwischen der Elektrodenanordnung 1 und diesen Gehäuse bei der Abgabe eines Defibrillationspulses aufgebautes elektrisches Feld verläuft dann durch das zu behandelnde Herz 9 hindurch.

Die erfindungsgemäß beanspruchte und durch die Ausführungsbeispiele illustrierte Elektrodenanordnung gestattet eine nichtinvasive Anpassung der Schockelektrodengeometrie eines ICD-Systems an patientenindividuelle anatomische Gegebenheiten. Dadurch wird eine universelle Einsetzbarkeit eines einzigen Elektrodentyps ermöglicht, was die Komplexität des Herstellungsprozesses der einzelnen Bauteile eines implantierbaren Kardioverter-Defibrillators deutlich reduziert und die Vorratshaltung in einem Krankenhaus erleichtert. Denn durch die erfindungsgemäß beanspruchte Vorrichtung ist es nicht mehr erforderlich, verschiedene, an unterschiedliche patientenindividuelle Anatomien angepasste Elektrodenanordnungen vorrätig zu halten. Vielmehr kann eine optimale Patientenversorgung mittels einer einzigen Elektrodenanordnung gewährleistet werden.

## Patentansprüche

1. Implantierbares medizinisches Gerät zur Stimulation eines menschlichen oder tierischen Herzens, mit einem Gehäuse, einer in dem Gehäuse angeordneten Stimulationseinheit zur Stimulation eines menschlichen oder tierischen Herzens (9), einer in dem Gehäuse angeordneten Detektionseinheit zur Detektion elektrischer Signale desselben Herzens (9) und einer ersten Elektrodenanordnung (1), die zumindest an die Stimulationseinheit angeschlossen ist, wobei die erste Elektrodenanordnung (1) einen ersten Elektrodenpol (2) aufweist, der zur Defibrillation des genannten Herzens (9) geeignet ist,
**dadurch gekennzeichnet,**
**dass** der erste Elektrodenpol (2) ein erstes Elektrodensegment (3) und mindestens ein weiteres Elektrodensegment (4, 11) aufweist, wobei die einzelnen Elektrodensegmente (3, 4, 11) gegeneinander elektrisch isoliert sind, wobei das mindestens eine weitere Elektrodensegment (4, 11) von der Stimulationseinheit zusammen mit dem ersten Elektrodensegment (3) oder unabhängig von dem ersten Elektrodensegment (3) mit einem Defibrillationspuls oder einem Stimulationspuls beaufschlagt werden kann.

2. Implantierbares medizinisches Gerät nach Anspruch 1, **dadurch gekennzeichnet, dass** zwischen benachbarten Elektrodensegmenten (3, 4, 11) des ersten Elektrodenpols (2) lediglich ein Isolator (5), jedoch kein anderer Elektrodenpol angeordnet ist.

3. Implantierbares medizinisches Gerät nach Anspruch 1 oder 2, **dadurch gekennzeichnet, dass** die einzelnen angesteuerten Elektrodensegmente (3, 4, 11) des ersten Elektrodenpols (2) während des Betriebs des implantierbaren medizinischen Geräts jeweils die gleiche Polarität aufweisen.

4. Implantierbares medizinisches Gerät nach einem der vorhergehenden Ansprüche,
**dadurch gekennzeichnet, dass** das implantierbare medizinische Gerät ein nicht-transvenös implantierbarer Kardioverter-Defibrillator ist.

5. Implantierbares medizinisches Gerät nach einem der vorhergehenden Ansprüche,
**dadurch gekennzeichnet, dass** das erste Elektrodensegment (3) eine größere Länge (L1) als das mindestens eine weitere Elektrodensegment (4) aufweist.

6. Implantierbares medizinisches Gerät nach einem der vorhergehenden Ansprüche,
**dadurch gekennzeichnet, dass** das implantierbare medizinische Gerät eine zweite Elektrodenanordnung aufweist, wobei die zweite Elektrodenanordnung einen zweiten Elektrodenpol aufweist, der als Gegenelektrode für den ersten Elektrodenpol (2) geeignet ist.

7. Implantierbares medizinisches Gerät nach Anspruch 6, **dadurch gekennzeichnet, dass** der zweite Elektrodenpol ein erstes Elektrodensegment und mindestens ein weiteres Elektrodensegment aufweist, wobei die einzelnen Elektrodensegmente gegeneinander elektrisch isoliert sind, wobei das mindestens eine weitere Elektrodensegment zusammen mit dem ersten Elektrodensegment oder unabhängig von dem ersten Elektrodensegment als Gegenelektrode für den ersten Elektrodenpol (2) der ersten Elektrodenanordnung (1) dienen kann.

8. Implantierbares medizinisches Gerät nach Anspruch 6 oder 7, **dadurch gekennzeichnet, dass** die erste Elektrodenanordnung (1) und die zweite Elektrodenanordnung baugleich ausgestaltet sind.

9. Implantierbares medizinisches Gerät nach einem der vorhergehenden Ansprüche,
**dadurch gekennzeichnet, dass** die erste Elektrodenanordnung (1) einen weiteren Elektrodenpol aufweist, der proximal oder distal von dem ersten Elektrodenpol (2) angeordnet ist.

10. Implantierbares medizinisches Gerät nach Anspruch 9, **dadurch gekennzeichnet, dass** der weitere Elektrodenpol zur Detektion elektrischer Signale des genannten Herzens (9) dient.

11. Elektrodenanordnung für ein implantierbares medizinisches Gerät zur Stimulation eines menschlichen oder tierischen Herzens (9), wobei die Elektrodenanordnung (1) einen ersten Elektrodenpol (2) aufweist, der zur Defibrillation des genannten Herzens (9) geeignet ist,
**dadurch gekennzeichnet,**
**dass** der erste Elektrodenpol (2) ein erstes Elektrodensegment (3) und mindestens ein weiteres Elektrodensegment (4, 11) aufweist, wobei die einzelnen Elektrodensegmente (3, 4, 11) gegeneinander elektrisch isoliert sind, wobei das mindestens eine weitere Elektrodensegment (4, 11) von einer Stimulationseinheit eines implantierbaren medizinischen Geräts zusammen mit dem ersten Elektrodensegment (3) oder unabhängig von dem ersten Elektrodensegment (3) mit einem Defibrillationspuls oder einem Stimulationspuls beaufschlagt werden kann.

12. Verfahren zur Steuerung des Betriebs eines implantierbaren medizinischen Geräts nach einem der Ansprüche 1 bis 10, wobei das implantierbare medizinische Gerät ein Gehäuse, eine in dem Gehäuse angeordnete Stimulationseinheit zur Stimulation eines menschlichen oder tierischen Herzens (9), eine in dem Gehäuse angeordnete Detektionseinheit zur Detektion elektrischer Signale desselben Herzens (9) und eine erste Elektrodenanordnung (1), die zumindest an die Stimulationseinheit angeschlossen ist, aufweist, wobei die erste Elektrodenanordnung (1) einen ersten Elektrodenpol (2) aufweist, der zur Defibrillation des genannten Herzens (9) geeignet ist, wobei der erste Elektrodenpol (2) ein erstes Elektrodensegment (3) und mindestens ein weiteres Elektrodensegment (4, 11) aufweist, wobei die einzelnen Elektrodensegmente (3, 4, 11) gegeneinander elektrisch isoliert sind,
**dadurch gekennzeichnet,**
**dass** das Verfahren folgende Schritte aufweist:
a) Vorgeben, welche Elektrodensegmente (3, 4, 11) im Fall einer gewünschten Defibrillation oder einer gewünschten Stimulation des genannten Herzens (9) eingesetzt werden sollen,
b) Verschalten der mittels der Vorgabe gemäß Schritt a) ausgewählten Elektrodensegmente (3, 4, 11), so dass die Stimulationseinheit die ausgewählten Elektrodensegmente (3, 4, 11) mit einem Stimulationspuls beaufschlagen kann,
c) Beibehalten der Vorgabe gemäß Schritt a) und des Verschaltens gemäß Schritt b), bis eine neue Vorgabe, welche Elektrodensegmente (3, 4, 11) im Fall einer gewünschten Defibrillation oder Stimulation des genannten Herzens (9) eingesetzt werden sollen, erfolgt.

13. Verfahren nach Anspruch 12, **dadurch gekennzeichnet, dass** das Verschalten gemäß Schritt b) elektronisch durchgeführt wird.

14. Verfahren nach Anspruch 12 oder 13, **dadurch gekennzeichnet, dass** das Verschalten gemäß Schritt b) ferngesteuert durchgeführt wird.

15. Verfahren zur Behandlung eines Patienten, der eine solche Behandlung benötigt, mittels eines implantierbaren medizinischen Geräts nach einem der Ansprüche 1 bis 10, wobei das implantierbare medizinische Gerät ein Gehäuse, eine in dem Gehäuse angeordnete Stimulationseinheit zur Stimulation des Herzens (9) des Patienten, eine in dem Gehäuse angeordnete Detektionseinheit zur Detektion elektrischer Signale des Herzens (9) des Patienten und eine erste Elektrodenanordnung (1), die zumindest an die Stimulationseinheit angeschlossen ist, aufweist, wobei die erste Elektrodenanordnung (1) einen ersten Elektrodenpol (2) aufweist, der zur Defibrillation des Herzens (9) des Patienten geeignet ist, wobei der erste Elektrodenpol (2) ein erstes Elektrodensegment (3) und mindestens ein weiteres Elektrodensegment (4, 11) aufweist, wobei die einzelnen Elektrodensegmente (3, 4, 11) gegeneinander elektrisch isoliert sind,
**dadurch gekennzeichnet,**
**dass** das Verfahren folgende Schritte aufweist:
a) Vorgeben, welche Elektrodensegmente (3, 4, 11) im Fall einer gewünschten Defibrillation oder einer gewünschten Stimulation des Herzens (9) des Patienten eingesetzt werden sollen,
b) Verschalten der mittels der Vorgabe gemäß Schritt a) ausgewählten Elektrodensegmente (3, 4, 11), so dass die Stimulationseinheit die ausgewählten Elektrodensegmente (3, 4, 11) mit einem Stimulationspuls beaufschlagen kann,
c) Zuführen eines Defibrillationspulses oder eines Stimulationspulses an alle ausgewählten und verschalteten Elektrodensegmente (3, 4, 11), um das Herz (9) des Patienten über alle ausgewählten und verschalteten Elektrodensegmente (3, 4, 11) mit diesem Defibrillationspuls oder Stimulationspuls zu beaufschlagen.
